# EUROPEAN PATENT APPLICATION

(11) **EP 3 952 298 A1**
(43) Date of publication of application: **09.02.2022**
(21) Application number: 21192505.2
(22) Date of filing: 11.07.2014
(51) Int. Cl.: H04N 5/74, A61B 10/00, A61B 90/00, G03B 21/14, G06T 1/00, G09G 5/00, G09G 5/36, H04N 7/18, A61B 5/06, A61B 5/00, G03B 17/54

(54) **PROJECTION SYSTEM**

(30) Priority: 14.11.2013 JP 2013235670
(62) Divisional of application: 14862613.8
(71) Applicant: Panasonic Intellectual Property Management Co., Ltd., Osaka-shi, Osaka 540-6207 (JP)
(72) Inventor: NAKAMURA, Masaaki, Osaka, 540-6207 (JP); MIMA, Kunihiro, Osaka, 540-6207 (JP)
(74) Representative: Eisenführ Speiser

(57) **Abstract**

Amedical operation support system 100A includes the light source 11, the imaging unit 12, the control unit 2, and the projecting unit 13. The light source 11 applies light of a predetermined wavelength band including the excitation light to the affected part 105. The imaging unit 12 captures a fluorescent image resulting from the fluorescence emission of the affected part 105. The control unit 2 generates the image data for projection based on the fluorescent image captured by the imaging unit 12. The projecting unit 13 projects the projection image based on the image data for projection onto the affected part 105 with visible light. In the medical operation support system 100A, the imaging optical axis Z2 of light incident on the imaging unit 12 is adjusted to be matched with the projection optical axis Z3' of light exiting from the projecting unit 13.

## Description

### TECHNICAL FIELD

The present disclosure relates to a projection system projecting a captured image of a subject onto a surface of the subject.

### BACKGROUND

PTL 1 discloses a surgical operation support system that outputs image data showing an affected part of a living body undergoing a surgical operation from a fluorescent imaging apparatus, and reproduces an image based on the image data using an image projection apparatus to display the image onto an actual affected part. To the affected part of the living body, a substance is preliminarily administered that emits fluorescence when light of a predetermined wavelength is applied. Therefore, this system projects an image acquired by capturing a fluorescence-emitting affected part onto the actual affected part to support identification of a lesioned part.

### CITATION LIST

### PATENT LITERATURE

PTL 1: Japanese Laid-Open Patent Publication No. 9-24053

### SUMMARY OF THE INVENTION

### PROBLEM TO BE SOLVED BY THE INVENTION

It is important for such a system to accurately project a projected image of a subject onto the actual subject.

An object of the present disclosure is to provide a projection system, which captures an image of a subject to project the image onto the subject, capable of reducing a positional deviation between the actual subject and the projected image.

### MEANS FOR SOLVING PROBLEM

A projection system according to the present disclosure includes an excitation light source, an imaging unit, a control unit, and a projecting unit. The excitation light source applies to a subject light of a predetermined wavelength band including excitation light. The imaging unit captures a fluorescent image resulting from fluorescence emission of the subject. The control unit generates image data for projection based on the fluorescent image captured by the imaging unit. The projecting unit projects a projection image based on the image data for projection onto the subject with visible light. In the projection system, an optical axis of light incident on the imaging unit is adjusted to be matched with an optical axis of light exiting from the projecting unit.

### EFFECT OF THE INVENTION

The projection system according to the present disclosure matches an optical path of light incident on the imaging unit with an optical path of light exiting from the projecting unit on the subject, and therefore can reduce a positional deviation between the actual subject and the projected image.

### BRIEF DESCRIPTION OF DRAWINGS

Fig. 1 is a schematic diagram showing a configuration of a medical operation support system according to a first embodiment.
Fig. 2A is a diagram showing an example of image data of a catheter stored in a memory.
Fig. 2B is a diagram showing an example of image data of a forceps stored in the memory.
Fig. 2C is a diagram showing an example of image data of a MERCI stored in the memory.
Fig. 3 is a diagram showing examples of image data of variations of the forceps.
Fig. 4 is a flowchart for explaining a projection image generation process in the medical operation support system according to the first embodiment.
Figs. 5A and 5B are explanatory diagrams of a state of an operative field in the medical operation support system according to the first embodiment.
Fig. 6 is a flowchart for explaining the projection image generation process in a modification of the first embodiment.
Fig. 7A, 7B and 7C are explanatory diagrams of states of an operative field in the medical operation support system according to the modification of the first embodiment.
Fig. 8 is a schematic diagram showing a configuration of a medical operation support system according to a second embodiment.
Fig. 9 is a schematic diagram showing a configuration of the medical operation support system according to a modification of the second embodiment.
Fig. 10 is a schematic diagram showing a configuration of a medical operation support system according to a third embodiment.
Fig. 11 is a diagram for explaining filtered wavelength bands of various lights of the medical operation support system according to the third embodiment.
Fig. 12 is a schematic diagram showing a configuration of a medical operation support system according to a fourth embodiment.
Fig. 13 is a diagram for explaining filtered wavelength bands of various lights of the medical operation support system according to the fourth embodiment.

### DESCRIPTION OF EMBODIMENTS

Hereinafter, embodiments will be described in detail with reference to the drawings as needed. It is noted that unnecessarily detailed description may not be made. For example, already well-known items may not be described in detail and substantially the same constituent elements may not repeatedly be described. This is because of avoiding unnecessary redundancy of the following description to facilitate the understanding by those skilled in the art.

It is noted that the accompanying drawings and the following description are provided by the applicant for sufficient understanding of the present disclosure by those skilled and are not intended to limit the subject described in the claims.

The following embodiments will be described by taking a medical operation support system used in medical setting as an example of a projection system.

### (First Embodiment)

### 1-1. Configuration

### 1-1-1. Overview of medical operation support system

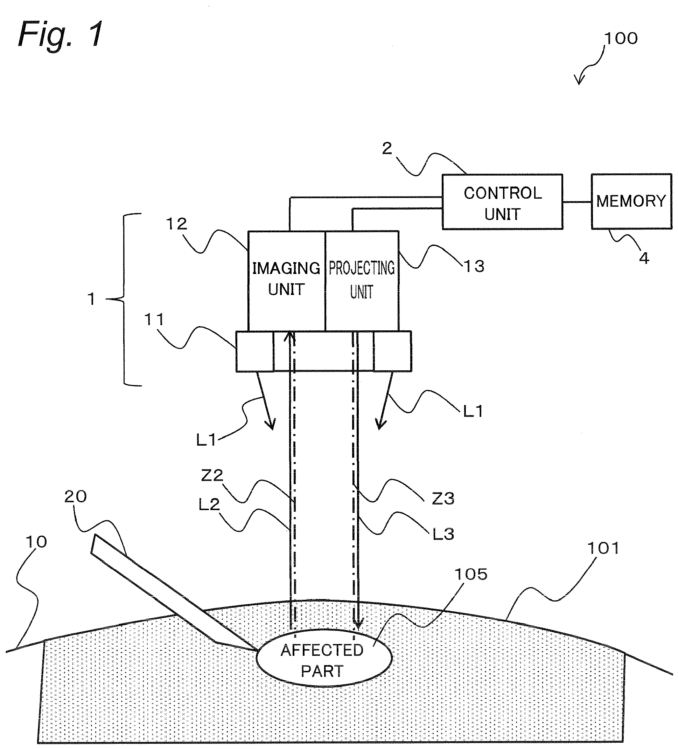

Fig. 1 is a schematic diagram showing a configuration of a medical operation support system according to a first embodiment. The medical operation support system is an example of a projection system.

The medical operation support system of this embodiment captures a fluorescent image of an affected part in an operative field, detects a fluorescence-emitting region of the affected part from the captured image, and projects a projection image with visible light onto a region of a subject corresponding to the detected region. As a result, a user (such as a doctor) of this medical operation support system can visually recognize a position or the like of an affected part of a patient.

While surgical operation is performed, a medical device such as a tube and a catheter is inserted into the patient's body. Normally, the medical device inserted into the patient's body cannot be visually recognized from the outside. The medical operation support system of this embodiment projects an image representing the shape of the medical device on a surface of a region in which the medical device is inserted. This enables a doctor or the like to recognize the position or the like of the medical device inserted into the patient's body.

In this situation, there exists a problem that if a region of the projection image projected on the operative field deviates from a region of the subject such as the fluorescence-emitting affected part and the medical device in the operative field, the positions of the affected part or the like are incorrectly recognized. To this end, the medical operation support system of this embodiment sets an optical axis of light incident on the imaging unit in parallel with an optical axis of light exiting from the projecting unit. As a result, the deviation is reduced between the fluorescence-emitting region and the region of the projection image. Therefore, a doctor or the like can correctly recognize the patient's affected part or the like.

### 1-1-2. Configuration of medical operation support system

A configuration of a medical operation support system 100 will hereinafter be described in detail.

The medical operation support system 100 includes an imaging/projecting apparatus 1, a control unit 2, and a memory 4. The imaging/projecting apparatus 1 includes a light source 11, an imaging unit 12, and a projecting unit 13.

As shown in Fig. 1, in an operative field 101 of a patient 10 undergoing surgery, there are an affected part 105 and a medical device 20. The imaging unit 12 of the imaging/projecting apparatus 1 captures the operative field 101. The control unit 2 controls every unit in the medical operation support system 100. Based on image data (captured image data) indicating an image captured by the imaging/projecting apparatus 1, the control unit 2 generates image data for projection of a projection image representing shapes of the affected part 105 or the medical device. The projecting unit 13 generates the projection image based on the image data for projection to project the projection image onto the operative field 101.

The memory 4 stores shape information. The control unit 2 reads the shape information from the memory 4 to execute a predetermined process. The shape information is image data indicating the shape of the medical device. Details of the shape information will be described later. The memory 4 is an example of a storage unit. For the memory 4, for example, a non-volatile memory or an HDD (Hard Disk Drive) can be used.

A photosensitive substance, which emits fluorescence when being excited by light (excitation light) of a predetermined wavelength, is in advance administered to the patient 10 undergoing surgery in the blood, lymph fluid, etc.. The photosensitive substance is accumulated in the affected part 105 where flows of the blood and lymph fluid are blocked therein. The photosensitive substance is a substance excited to emit fluorescence when near-infrared light is applied, for example, and is indocyanine green, for example. The affected part 105 with the photosensitive substance accumulated emits fluorescence when the excitation light is applied from the light source 11.

The photosensitive substance to emit fluorescence responding to excitation is applied to a surface of, or kneaded into, the medical device 20 used in the surgery.

The light source 11 applies the excitation light having a wavelength within an excitation wavelength range of the photosensitive substance. For example, the light source 11 applies the light of a near-infrared wavelength band. The light source 11 is an example of an excitation light source. The light source 11 is disposed to surround the periphery of the imaging unit 12 and the projecting unit 13.

The imaging unit 12 includes, for example, a high-sensitivity CCD camera. The imaging unit 12 includes therein an imaging optical system having a lens or the like. The imaging unit 12 captures a fluorescent image resulting from the fluorescence emission of the photosensitive substance of the affected part 105 and the medical device 20 to generate captured image data. The captured image data is image data indicating a fluorescent image of the fluorescence-emitting region and is input to the control unit 2. The imaging unit 12 may capture a visible light image of the operative field 101 together with the fluorescent image. The imaging unit 12 may include a camera group that is a combination of cameras capable of detecting one or more types of lights among visible light, fluorescent light, and excitation light, for example, so as to detect all the types of the lights described above. In this embodiment, the imaging unit 12 generates the captured image data indicating the fluorescent image. The imaging unit 12 may output to the control unit 2 the data of all the images of captured results including the fluorescent image and the visible light image. In this case, the control unit 2 may extract the fluorescent image from the entire image of the captured results.

The control unit 2 has a function of controlling the operation of the imaging/projecting apparatus 1. The control unit 2 acquires the captured image data from the imaging unit 12 and generates the image data for projection indicating the shapes of the affected part 105 and the medical device based on the captured image data.

When projecting a projection image representing the shape of the medical device, the control unit 2 reads the shape information (described in detail later) from the memory 4 and generates the image data for projection. When projecting a projection image representing the shape of the affected part 105, the control unit 2 generates the image data for projection indicating the fluorescent image of the affected part 105 in the captured image data. Based on the shape and the wavelength band of the fluorescent image in the captured image data, the control unit 2 discriminates between the affected part 105 and the medical device 20 (described in detail later).

The control unit 2 outputs the generated image data for projection to the projecting unit 13. The control unit 2 includes a CPU or an MPU, for example, and implements the function thereof by executing a predetermined program. The function of the control unit 2 may be implemented by a dedicated electronic circuit.

The projecting unit 13 generates a projection image based on the image data from the control unit 2 and projects the projection image onto a surface of the affected part in which the medical device 20 is inserted. The projecting unit 13 includes, for example, a projector. The projecting unit 13 includes therein a projection optical system having a lens or the like.

In this embodiment, the optical axis of light incident on the imaging unit 12 is referred to as an "imaging optical axis". An imaging optical axis Z2 is defined by the imaging optical system of the imaging unit 12. Further, the optical axis of light exiting from the projecting unit 13 is referred to as a "projection optical axis". A projection optical axis Z3 is defined by projection optical system of the projecting unit 13. As shown in Fig. 1, the imaging unit 12 and the projecting unit 13 are adjacently arranged in parallel above the affected part 105 such that the imaging optical axis Z2 and the projection optical axis Z3 are made parallel to each other. As a result, a positional deviation can be reduced between a region in the operative field 101 captured by the imaging unit 12 and a region of projection of the projection image by the projecting unit 13. The light source 11 is disposed to surround both of the imaging optical axis Z2 and the projection optical axis Z3.

The memory 4 is connected to the control unit 2 so that the control unit 2 can read the shape information to execute a process described later. The shape information is image data indicative of the shape of the medical device. The shape information is not limited to the shape of the medical device and may be image data of an object having a predetermined shape.

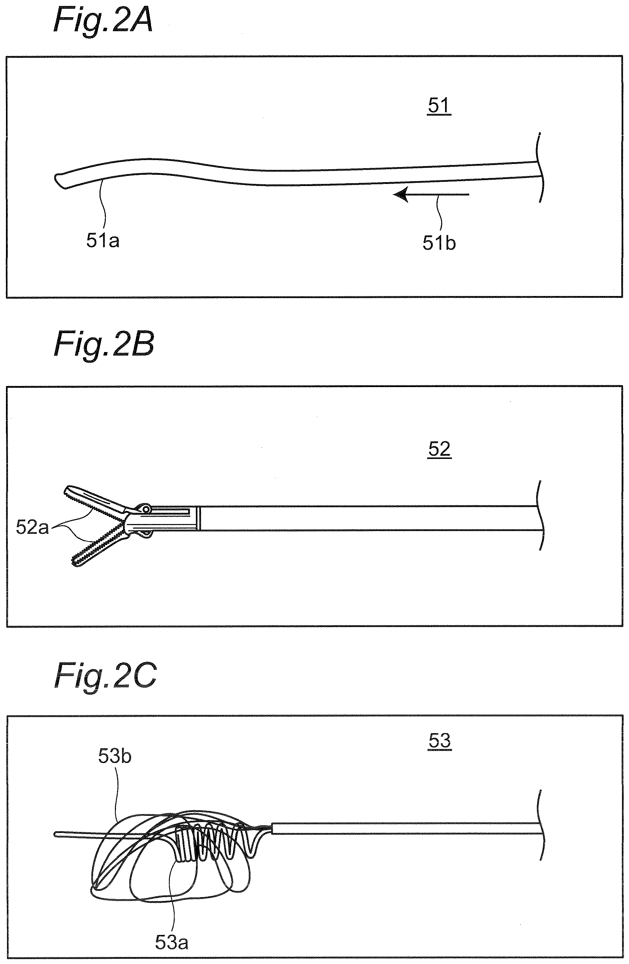

Figs. 2A to 2C show examples for respective pieces of the shape information of a plurality of types for surgical instruments stored in the memory 4. Fig. 2A shows image data 51 of a catheter, Fig. 2B shows image data 52 of a forceps, and Fig. 2C shows image data 53 of a MERCI retriever (hereinafter referred to as a MERCI) . The image data 51 to 53 are examples of the shape information of the respective surgical instruments.

The catheter is a tubular surgical instrument inserted into the body of the patient 10. One of the characteristics of the image data 51 of the catheter is that the catheter extends in a longitudinal direction 51b to a tip 51a with a constant width.

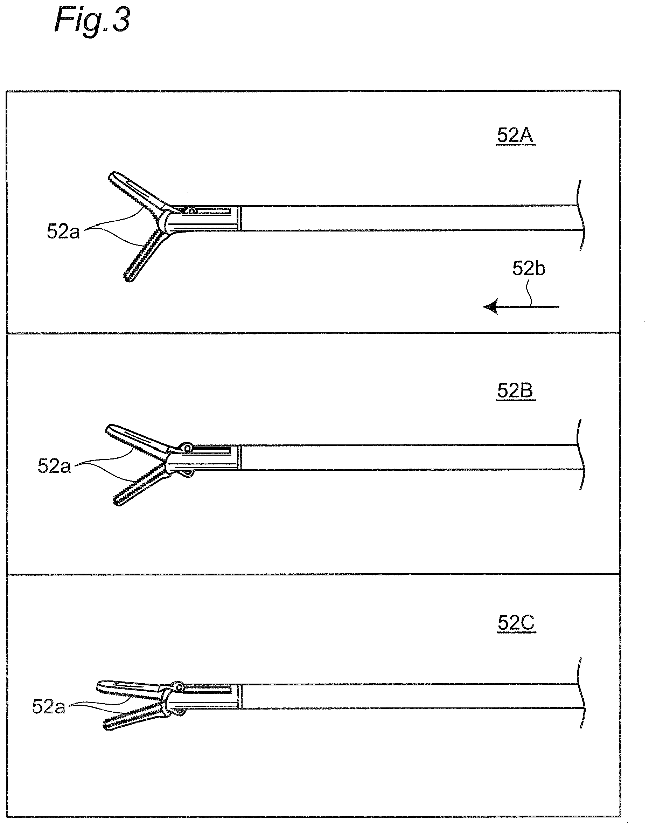

The forceps is a surgical instrument used for holding and pulling an affected part and a suture thread. One of the characteristics of the image data 52 of the forceps is a forked holding portion 52a disposed at a tip thereof. Fig. 3 shows examples for the image data of variations of the forceps. In image data 52A to 52C of the forceps, the holding portion 52a is opened at different angles respectively. The memory 4 stores as the shape information the image data of variations of the forceps such as the image data 52A to 52C.

The MERCI is a surgical instrument inserted into a blood vessel or the like for removing a blood clot and has a loop wire and a filament for entwining and removing a blood clot. One of the characteristics of the image data 53 of the MERCI is a helical loop wire 53a disposed at a tip thereof. The image data 53 of the MERCI has a threadlike filament 53b.

If the medical operation support system 100 does not project the projection image representing the shape of the medical device, for example, when only the projection image representing the shape of the affected part 105 is projected, the medical operation support system 100 may not include the memory 4.

### 1-2. Operation

The operation of the medical operation support system 100 configured as described above will hereinafter be described.

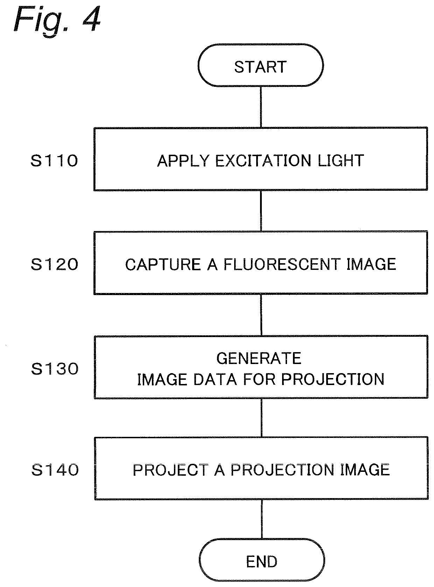

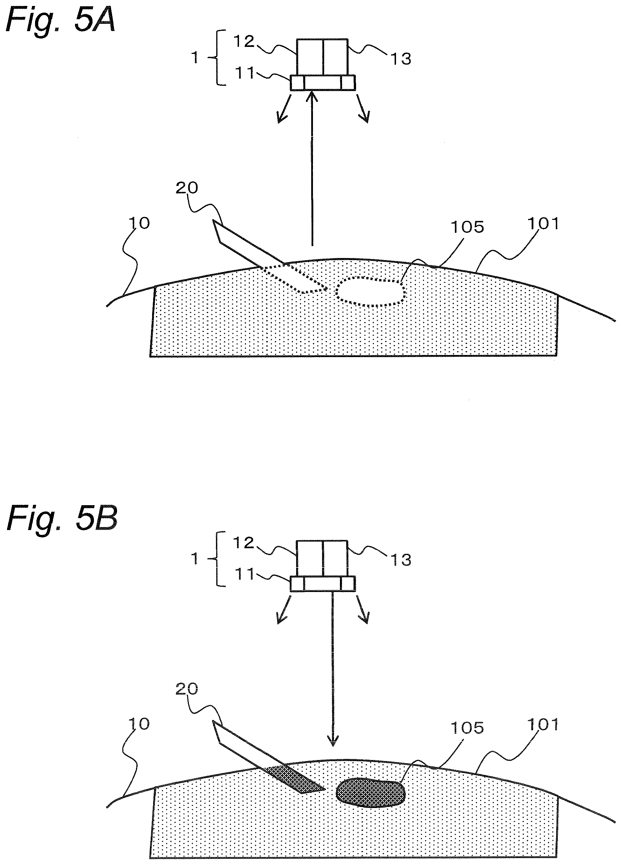

Fig. 4 is a flowchart for explaining a projection image generation process in the medical operation support system 100. Fig. 5A is an explanatory diagram of a state of an operative field in the medical operation support system 100. Fig. 5B is an explanatory diagram of a state in which the projection image generation process is executed for the operative field of Fig. 5A.

First, the control unit 2 drives the light source 11 to apply the excitation light to the operative field 101 including the affected part 105 (step S110). The excitation light from the light source 11 excites the photosensitive substance of the affected part 105 of the patient 10 and the medical device 20, so that the affected part 105 and the medical device 20 emit fluorescence as shown in Fig. 5A.

Subsequently, the control unit 2 controls the imaging/projecting apparatus 1 to capture the operative field 101 with the imaging unit 12 (step S120). In this case, the imaging unit 12 generates the captured image data indicating the fluorescent image resulting from the photosensitive substance of the affected part 105 and the medical device 20. The captured image data is output to the control unit 2.

The control unit 2 generates image data for projection based on the captured image data (step S130) . Based on the fluorescent image in the captured image data, the control unit 2 generates the image data for projection for displaying the projection image representing the shape of the fluorescent image with visible light. The control unit 2 outputs the image data for projection to the projecting unit 13.

In this regard, first, the control unit 2 determines whether an object to which a projection image is projected is an affected part 105 or a medical device 20 based on the shape of the fluorescent image in the captured image data. The control unit 2 may determine whether the object is the affected part 105 or the medical device 20 based on the wavelength band of the fluorescent image in the captured image data, for example. In this case, photosensitive substances emitting fluorescence in different wavelength bands are contained in the affected part 105 and the medical device 20 in advance.

If the affected part 105 is the object to which the projection image is projected, the control unit 2 generates the image data for projection indicating the shape of the fluorescent image itself in the captured image data.

If the medical device 20 is the object to which the projection image is projected, the control unit 2 selects the shape information based on similarity to the fluorescent image, from a plurality of pieces of the shape information stored in the memory 4, and generates the image data for projection representing the shape indicated by the shape information. For example, the control unit 2 selects the image data most similar to the fluorescent image of the captured image data out of the image data 51 to 53 of the surgical instrument shown in Figs. 2A to 2C, and causes the projecting unit 13 to project the image.

The projecting unit 13 projects the projection image based on the image data for projection to the fluorescence-emitting region on the surface of the affected part 105 in the operative field 101 (step S140) . The projection image enables a doctor or the like to clearly visually recognize the positions and shapes of the affected part 105 and the medical device 20 in the operative field 101 as shown in Fig. 5B.

The process described above is repeatedly executed in predetermined cycles (e.g., 1/60 second). As a result, an image is captured and projected every 1/60 seconds for example, and a user therefore can visually recognize the position and shape of the affected part 105 as a real-time moving image.

Optical paths of various lights used in the process described above will be described with reference to Fig. 1.

In Fig. 1, the imaging unit 12 and the projecting unit 13 are adjacently arranged in parallel above the affected part 105. The light source 11 is disposed to surround both the imaging optical axis Z1 of the imaging unit 12 and the projection optical axis L3 of the projecting unit 13, and applies an excitation light L1.

The fluorescent light emitted from the photosensitive substance accumulated in the affected part 105 due to the excitation light L1 applied from the light source 11 is incident on the imaging unit 12 through an optical path (hereinafter, referred to as an "imaging optical path") L2 along the imaging optical axis Z2. The projection image from the projection apparatus 3 is projected onto the affected part 105 through an optical path (hereinafter, referred to as a "projection optical path") L3 along the projection optical axis Z3. In this state, the imaging optical axis Z2 and the projection optical axis Z3 are configured to be parallel to each other and, therefore, the imaging optical path L2 and the projection optical path L3 are parallel to each other.

If at least one of the imaging unit 12 and the projecting unit 13 is disposed obliquely with respect to the affected part 105 (subject), a deviation inevitably occurs between the affected part 105 and the projection image. In contrast, since the imaging unit 12 and the projecting unit 13 are arranged such that the imaging optical axis Z2 of the imaging unit 12 and the projection optical axis Z3 of the projecting unit 13 are made parallel to each other as shown in Fig. 1, the imaging optical path L2 and the projection optical path L3 are made parallel to each other and the deviation is reduced between the affected part 105 and the projection image.

### 1-3. Effect and the like

As described above, in this embodiment, the medical operation support system 100 includes the light source 11, the imaging unit 12, the control unit 2, and the projecting unit 13. The light source 11 applies light of a predetermined wavelength band including the excitation light to the affected part 105 emitting fluorescence in response to the excitation light. The imaging unit 12 captures a fluorescent image resulting from the fluorescence emission of the affected part 105. The control unit 2 generates the image data for projection based on the fluorescent image captured by the imaging unit 12. The projecting unit 13 projects the projection image based on the image data for projection onto the affected part 105 with visible light. The imaging optical axis Z2 and the projection optical axis Z3 are set to be parallel to each other.

The above configuration makes the imaging optical path L2 along the imaging optical axis Z2 and the projection optical path L3 along the projection optical axis Z3 parallel to each other, so that the deviation can be reduced between the region on which the affected part 105 emits fluorescence and the region on which the projection image is projected.

### 1-3-1. Modification

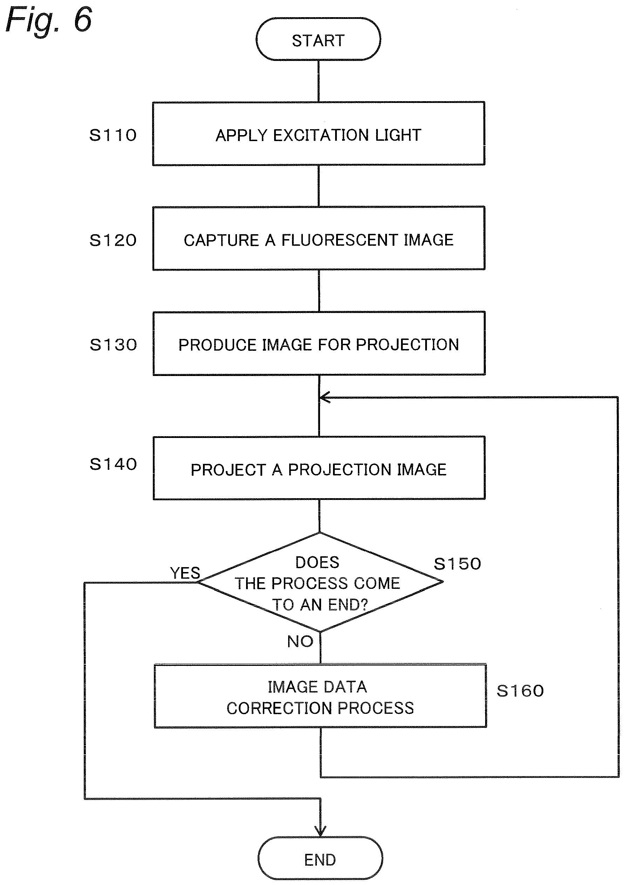

Fig. 6 shows a modification of the projection image generation process. In the projection image generation process, this medical operation support system 100 may detect the projection image projected from the projecting unit 13 and correct the image data for projection so as to correct a positional deviation of a new projection image.

In a flowchart shown in Fig. 6, after the projection image is projected from the projecting unit 13 (step S140), the control unit 2 executes an image data correction process (step S160). The image data correction process is a process of detecting the projection image actually projected onto the operative field 101 and the fluorescent image to correct the image data for projection in accordance with a difference between the projection image and the fluorescent image.

The corrected image data for projection is output again to the projecting unit 13. The projecting unit 13 projects a projection image based on the corrected image data for projection onto the affected part 105 of the operative field 101 (step S140). Until an operation for termination is performed (step S150), the control unit 2 repeatedly executes the correction of the image data for projection (step S160).

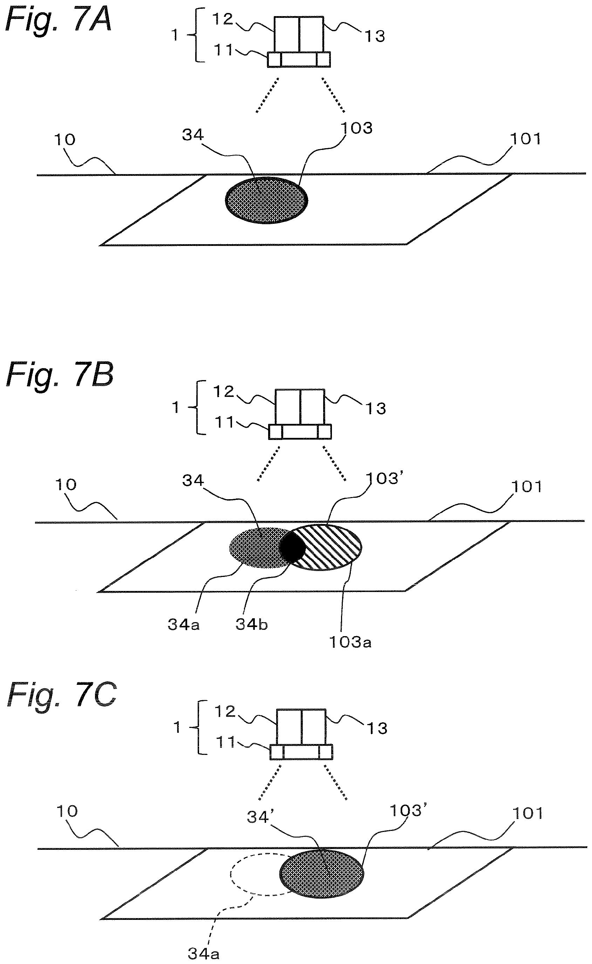

Description will be made of the image data correction process in the case that an affected part moves over time with reference to Figs. 7A-7C.

Fig. 7A shows a state in which the projection image 34 is projected while the position of the projection image 34 from the projecting unit 13 is coincident with the position of an affected part 105. Fig. 7B shows a state in which the affected part 103 has moved from the state of the operative field 101 shown in Fig. 7A. Since the affected part 103 has moved, a deviation occurs as shown in Fig. 7B between a fluorescence-emitting region of an affected part 103' after the movement and the projection region of the projection image 34 from the projecting unit 13. At this time, the control unit 2 of the medical operation support system 100 executes the above image data correction process as follows.

First, the control unit 2 detects a region 103a that is a region of the affected part 103 without display of the projection image 34 and corrects the image data for projection such that the projection image is displayed in the region 103a.

The control unit 2 detects a region 34a that is a region without fluorescence emission with the projection image 34 displayed due to the deviation, and corrects the image data for projection so as not to display the projection image 34 in the region 34a.

As described above, by detecting the projection image actually projected onto the operative field 101 and the fluorescent image to correct the image data for projection in accordance with the difference between the projection image and the fluorescent image, the affected part 103' after the movement can be matched with a projection image 34' as shown in Fig. 7C so that the deviation of the display by the projecting unit 13 can be eliminated. Therefore, even when a deviation occurs due to a surface shape or movement of an affected part, the image of the affected part can be projected at a proper position because of the image data correction process.

### (Second Embodiment)

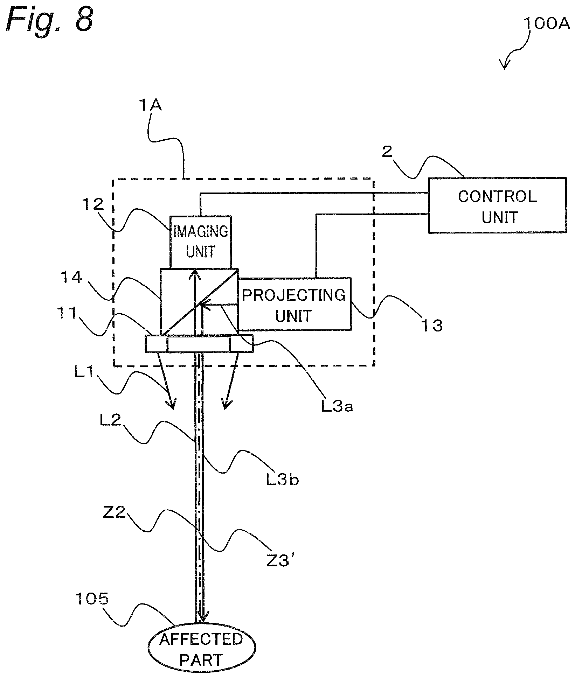

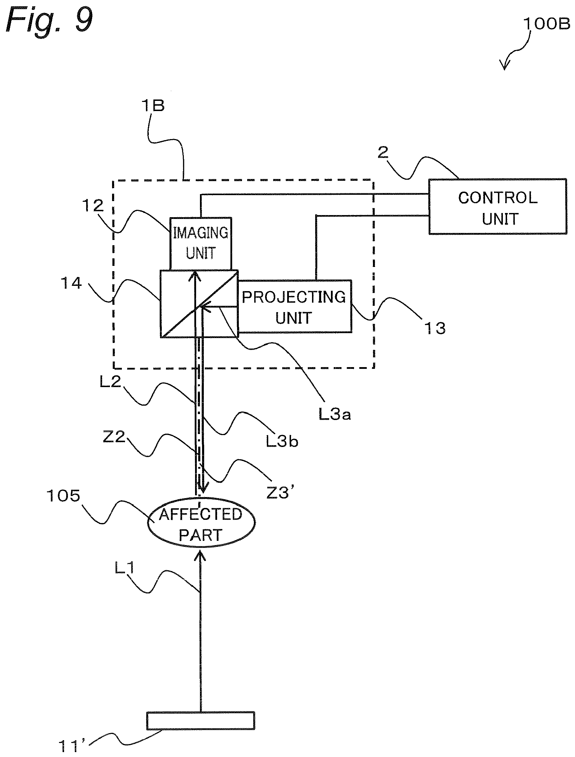

A medical operation support system according to a second embodiment will hereinafter be described with reference to Figs. 8 and 9. In the medical operation support system 100 according to the first embodiment, although the imaging optical path L2 of light incident on the imaging unit 12 and the projection optical path L3 of light exiting from the projecting unit 13 are parallel, the paths are not identical (see Fig. 1). Thus, even when the projection image has the shape of the fluorescent image same as the captured affected part (subject), a slight deviation occurs between the affected part and the projection image. In this regard, a medical operation support system 100A of this embodiment matches the optical axis of light incident on the imaging unit 12 with the optical axis of light exiting from the projecting unit 13, so as to further reduce the deviation between the affected part and the projection image due to the arrangement positions of the units.

The medical operation support system 100A will hereinafter be described without describing the same constituent elements and operations as those of the medical operation support system 100 according to the first embodiment when appropriate.

### 2-1. Configuration

Fig. 8 is a schematic diagram showing a configuration of the medical operation support system according to the second embodiment. The medical operation support system 100A includes an imaging/projecting apparatus 1A and the control unit 2. The imaging/projecting apparatus 1A includes the light source 11, the imaging unit 12, and the projecting unit 13 as is the case with the first embodiment and further includes a dichroic mirror portion 14.

The dichroic mirror portion 14 includes a dichroic mirror that transmits light of the same wavelength band as the fluorescence wavelength band of the fluorescent image captured by the imaging unit 12 and that reflects the visible light. For example, the dichroic mirror portion 14 includes a dichroic mirror transmitting the near-infrared light and reflecting the visible light. The dichroic mirror portion 14 bends the optical path of the visible light by 90° according to the reflection.

The imaging unit 12 and the projecting unit 13 are arranged in the directions orthogonal to each other via the dichroic mirror portion 14 such that the imaging optical axis Z2 and a projection optical axis Z3' are matched with each other. The projection optical axis Z3' is an optical axis of light exiting from the projecting unit 13, and is defined by the projection optical system of the projecting unit 13 and the dichroic mirror portion 14. The visible light from the projecting unit 13 is reflected by the dichroic mirror portion 14 so that the imaging optical axis Z2 and the projection optical axis Z3' are matched with each other. According to this, on the subject such as the affected part 105, the positional deviation can be eliminated between the region which the imaging unit 12 captures and the region on which the projecting unit 13 projects the projection image.

In the first embodiment, the light source 11 and the imaging unit 12 are adjacently arranged. In the imaging/projecting apparatus 1A of this embodiment, the dichroic mirror portion 14 is disposed between the light source 11 and the imaging unit 12. Accordingly, the imaging optical system (not shown) of the imaging unit 12 is configured to be incorporated inside the imaging unit 12 so as not to interfere with the dichroic mirror portion 14. The projecting unit 13 has the projection optical system (not shown) configured to be incorporated inside the projecting unit 13 so as to avoid interference with the dichroic mirror portion 14.

### 2-2. Operation

The operation of the medical operation support system 100A configured as described above will hereinafter be described.

The medical operation support system 100A of this embodiment executes the same projection image generation process as the first embodiment (see Fig. 4). Optical paths of various lights used in the projection image generation process of this embodiment will hereinafter be described.

As shown in Fig. 8, the excitation light L1, which is applied from the light source 11 disposed to surround both of the imaging optical axis Z2 of the imaging unit 12 and the projection optical axis Z3' of the projecting unit 13, excites the photosensitive substance accumulated in the affected part 105. Then, the excited photosensitive substance emits fluorescence.

The fluorescent light from the photosensitive substance accumulated in the affected part 105 goes straight inside the dichroic mirror portion 14 and is incident on the imaging unit 12 through the imaging optical path L2 along the imaging optical axis Z2.

On the other hand, the light exiting from the projecting unit 13 for projection of the projection image is incident on the dichroic mirror portion 14 through a projection optical path L3a orthogonal to the imaging optical axis Z2. Then, the light for projection of the projection image is changed in traveling direction by 90° due to reflection in the dichroic mirror portion 14, and exits from the dichroic mirror portion 14 along a projection optical path L3b. The projection optical path L3b is an optical path along the projection optical axis Z3'. Subsequently, the light for projection of the projection image goes through the projection optical path L3b and is projected onto the affected part 105.

Although Fig. 8 shows the imaging optical path L2 and the projection optical path L3b without overlap for the convenience of explanation, these optical paths are identical.

As described above, the imaging/projecting apparatus 1A of this embodiment has the imaging optical path L2 and the projection optical path L3b matched with each other. Therefore, no positional deviation occurs between the projection image, which has the shape of the fluorescent image same as the affected part 105 captured by the imaging unit 12, and the actual affected part 105 where the projection image is projected.

In the imaging/projecting apparatus 1A shown in Fig. 8, the arrangement positions of the imaging unit 12 and the projecting unit 13 may be replaced with each other. In this case, a dichroic mirror used in the dichroic mirror portion 14 reflects the light of the wavelength band of the fluorescence emission and transmits the visible light. The light incident on the imaging unit 12 is reflected by the dichroic mirror portion 14 to match the imaging optical axis with the projection optical axis. As a result, the same effect as described above can be provided.

Further, the dichroic mirror included in the dichroic mirror portion 14 may be a dichroic mirror transmitting only the light having the wavelength band of the fluorescence emitted by the photosensitive substance. Alternatively, a filter transmitting only the light having the wavelength band of the fluorescence emitted by the photosensitive substance may be disposed between the imaging unit 12 and the dichroic mirror portion 14. As a result, only the fluorescence emitted by the photosensitive substance forms an image in the imaging unit 12. This facilitates the control unit 2 to execute an image process for the image captured by the imaging unit 12.

### 2-3. Effect and the like

As described above, in this embodiment, the medical operation support system 100A includes the light source 11, the imaging unit 12, the control unit 2, and the projecting unit 13. The light source 11 applies light of a predetermined wavelength band including the excitation light to the affected part 105. The imaging unit 12 captures a fluorescent image resulting from the fluorescence emission of the affected part 105. The control unit 2 generates the image data for projection based on the fluorescent image captured by the imaging unit 12. The projecting unit 13 projects the projection image based on the image data for projection onto the affected part 105 with visible light. In the medical operation support system 100A, the imaging optical axis Z2 of light incident on the imaging unit 12 is adjusted to be matched with the projection optical axis Z3' of light exiting from the projecting unit 13.

Since the above configuration matches the imaging optical path L2 along the imaging optical axis Z2 with the projection optical path L3 along the projection optical axis Z3' on the affected part 105, the positional deviation can be reduced between the region on which the affected part 105 emits fluorescence and the region on which the projection image is projected.

### 2-3-1. Modification

In the second embodiment, the imaging/projecting apparatus is configured to dispose the light source adjacently to the imaging unit. However, the light source may not adjacently be disposed to the imaging unit. A modification of the second embodiment will hereinafter be described with reference to Fig. 9.

Fig. 9 is a schematic diagram showing a configuration of a medical operation support system according to the modification of the second embodiment. A medical operation support system 100B has a light source 11' disposed on the side opposite to an imaging/projecting apparatus 1B across the affected part 105.

The light source 11' is disposed at a position not surrounding the imaging optical axis Z2 and the second projection optical axis Z3'. Therefore, a light source such as a point light source and a surface light source suitable for applying the excitation light L1 to the affected part 105 can be selected as the light source 11'.

Instead of the configuration in which the imaging unit 12 captures the fluorescence generated by the photosensitive substance accumulated in the affected part 105 (subject) due to the excitation light L1, the medical operation support system 100B may be configured to capture the light applied from the light source 11' and passing through the affected part 105 without introducing the photosensitive substance into the affected part 105. In this case, the light source 11' may apply light (electromagnetic waves) such as x-rays and γ-rays as well as the infrared rays, visible light rays, and ultraviolet rays. The electromagnetic waves applied from the light source 11' may be electromagnetic waves in the frequency range except electric waves.

### (Third Embodiment)

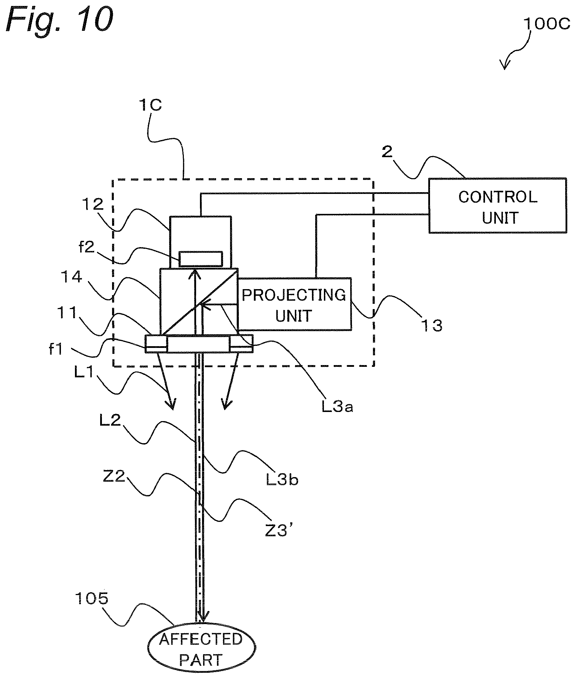

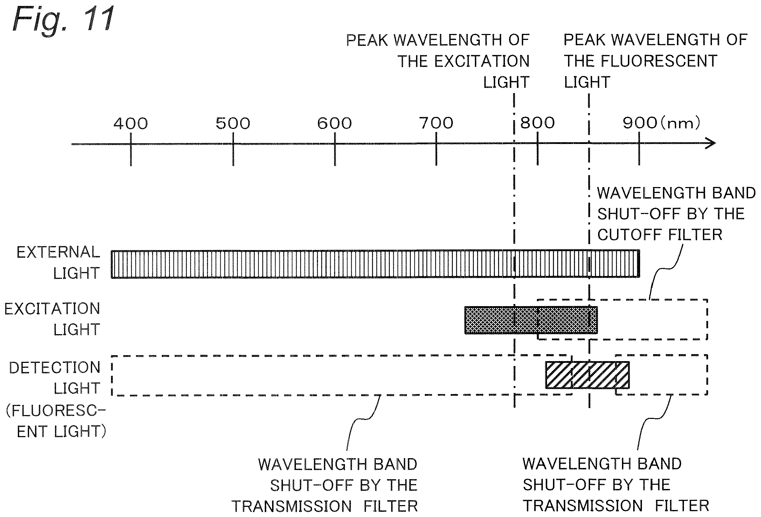

A third embodiment will hereinafter be described with reference to Figs. 10 and 11. In the second embodiment, the imaging/projecting apparatus is configured such that the imaging optical axis is matched with the projection optical axis . In this embodiment, additionally, the light incident on the imaging unit is limited in a certain wavelength band.

A medical operation support system 100C will hereinafter be described without describing the same constituent elements and operations as those of the first and second embodiments when appropriate.

### 3-1. Overview of medical operation support system

When the imaging unit detects a fluorescent image of an subject such as an affected part, the light other than the fluorescence emission of the subject generates detection noise due to a component of the same wavelength band as the fluorescence emission of the subject. Particularly, although the excitation light applied from the light source is necessary for causing the subject to emit fluorescence, the excitation light may generate the detection noise. In this regard, this embodiment includes a filter to shut off a wavelength band component that may generate the detection noise out of the light incident on the imaging unit and the light applied from the light source.

### 3-2. Configuration of medical operation support system

Fig. 10 is a schematic diagram showing a configuration of a medical operation support system according to the third embodiment. The medical operation support system 100C includes an imaging/projecting apparatus 1C and the control unit 2. The imaging/projecting apparatus 1C includes the light source 11, the imaging unit 12, and the projecting unit 13 as is the case with the imaging/projecting apparatus 1A of the second embodiment and further includes a cutoff filter (second filter) f1 and a transmission filter (first filter) f2.

The cutoff filter f1 is a film filter attached onto the light source 11. The cutoff filter f1 composes a short-pass filter (long-wavelength cut filter) to shut off the light applied from the light source 11 equal to or higher than a peak wavelength band of the fluorescence emission of the photosensitive substance contained in the subject such as the affected part 105. The cutoff filter f1 transmits a peak wavelength component of the excitation light less than the peak wavelength of the fluorescence emission of the affected part 105 out of the light applied from the light source 11.

The transmission filter f2 is a film filter built into the imaging unit 12. The transmission filter f2 composes a band pass filter transmitting a predetermined wavelength band component including the peak wavelength of the fluorescence emission of the affected part 105 or the like out of the light incident on the imaging unit 12. The wavelength band component transmitted by the transmission filter f2 does not contain the wavelength band of the visible light and is greater than the peak wavelength of the excitation light.

Fig. 11 is a diagram illustrating filtered wavelength bands of various lights of the medical operation support system according to this embodiment. Fig. 11 shows the wavelength bands of the fluorescent light and the excitation light of indocyanine green that is the photosensitive substance contained in the subject in this embodiment. The fluorescence of indocyanine green has the wavelength band of substantially 810 nm to 890 nm and the peak wavelength of substantially 850 nm. On the other hand, the excitation light of indocyanine green has the wavelength band of substantially 730 nm to 860 nm and the peak wavelength of substantially 780 nm. Therefore, the fluorescence and the excitation light of indocyanine green partially overlap in a certain wavelength band. If the excitation light in this overlapping wavelength band is applied to the affected part 105, discrimination cannot be made between the fluorescence emission of the affected part 105 and a reflected light of the excitation light on the surface of the affected part 105, resulting in detection noise.

In this regard, the medical operation support system 100C uses the cutoff filter f1 and the transmission filter f2 to filter the lights such that the overlapping wavelength band is separated between the light applied from the light source 11 (excitation light) and the light incident on the imaging unit 12 (detection light). Specifically, the cutoff filter f1 transmits the wavelength band component less than the wavelength of 800 nm and shuts off the wavelength band component of the wavelength of 800 nm or more out of the light applied from the light source 11 (excitation light). Further, the transmission filter f2 transmits the wavelength band component of the wavelength 820 nm to 880 nm and shuts off the wavelength band component of the wavelength smaller than 820 nm and that of the wavelength greater than 880 nm out of the light incident on the imaging unit 12.

As a result, the imaging unit 12 can be restrained from receiving the component of the excitation light in the wavelength band overlapping with the wavelength band of the fluorescence emission, so as to reduce the detection noise. Further, since the transmission filter f2 transmits the component of the peak wavelength of the fluorescence, the detection efficiency of the fluorescent image can be maintained. Since the cutoff filter f1 transmits the component of the peak wavelength of the excitation light, the efficiency of causing the affected part 105 to emit fluorescence can be maintained.

### 3-3. Effect and the like

As described above, in this embodiment, the medical operation support system 100C includes the light source 11, the imaging unit 12, the control unit 2, and the projecting unit 13. The light source 11 applies light of a predetermined wavelength band including the excitation light to the affected part 105. The imaging unit 12 captures a fluorescent image resulting from emission of fluorescent light from the affected part 105. The control unit 2 generates the image data for projection based on the fluorescent image captured by the imaging unit 12. The projecting unit 13 projects the projection image based on the image data for projection onto the affected part 105 with visible light. In the medical operation support system 100C, the imaging optical axis Z2 of light incident on the imaging unit 12 is adjusted to be matched with the projection optical axis Z3' of light exiting from the projecting unit 13.

The medical operation support system 100C further includes the transmission filter (first filter) f2 and the cutoff filter (second filter) f1. The transmission filter f2 cuts off a component of a predetermined wavelength band including the peak wavelength of the excitation light out of the light incident on the imaging unit 12. The fluorescence cutoff filter f1 cuts off a component of a predetermined wavelength band including the peak wavelength of the fluorescent light out of the light applied by the light source 11.

With the above configuration, the imaging unit 12 can be restrained from receiving the component of the excitation light in the wavelength band overlapping with the wavelength band of the fluorescence emission so as to reduce the detection noise when the imaging unit 12 detects a fluorescent image of a subject such as an affected part.

Additionally, even when light of a wavelength band other than the fluorescence emission can be detected because of the characteristics of the imaging unit 12, since the transmission filter f2 transmits only the predetermined wavelength band component including the peak wavelength of the fluorescent light, the detection noise due to such device characteristics can be suppressed. Further, even when light of a wavelength band other than the excitation light is applied because of the characteristics of the light source 11, since the cutoff filter f1 transmits only the predetermined wavelength band component including the peak wavelength of the excitation light, the detection noise due to such device characteristics can be suppressed.

### (Fourth Embodiment)

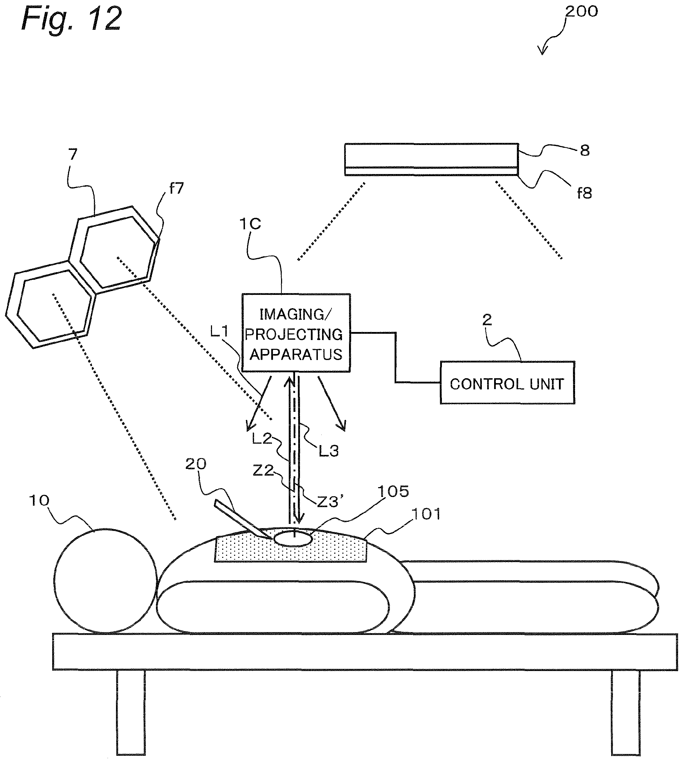

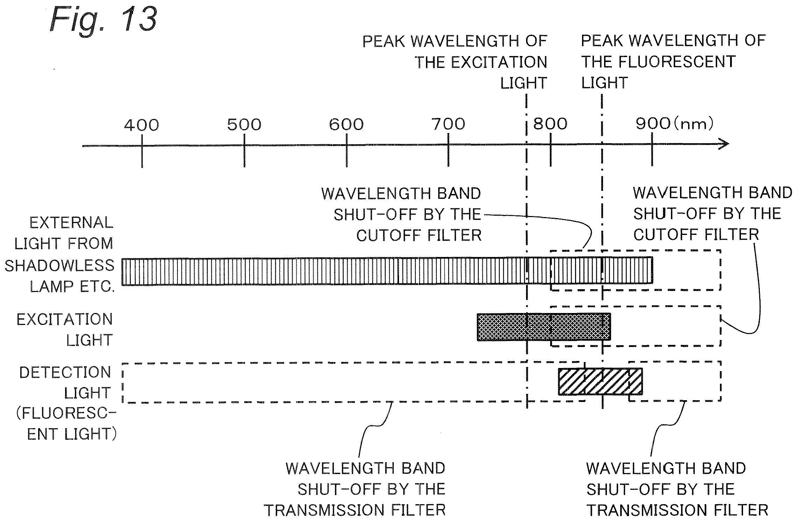

A fourth embodiment will hereinafter be described with reference to Figs. 12 and 13. In the third embodiment, the wavelength band components generating the detection noise are shut-off out of the light incident on the imaging unit and the light applied from the light source. In this embodiment, a wavelength band component generating the detection noise is further shut-off out of the external light.

A medical operation support system 200 will hereinafter be described without describing the same constituent elements and operations as those of the first to third embodiments when appropriate.

### 4-1. Overview of medical operation support system

When surgery is performed, an external light source (such as a fluorescent lamp and a shadowless lamp) is used for illuminating the operative field with visible light in addition to the light source applying the excitation light. As shown in Fig. 11, the external light from the external light source generally includes a wavelength band component other than the visible light region, leading to generation of detection noise. Therefore, in this embodiment, the wavelength band component generating the detection noise is shut-off out of the external light.

### 4-2. Configuration of medical operation support system

Fig. 12 is a schematic diagram showing a configuration of a medical operation support system according to the fourth embodiment.

The medical operation support system 200 of this embodiment includes the imaging/projecting apparatus 1C and the control unit 2 as is the case with the third embodiment and further includes cutoff filters (third filters) f7, f8. A shadowless lamp 7 and a fluorescent lamp 8 are disposed around the medical operation support system 200.

The shadowless lamp 7 is a lighting device configured to diffusely reflect light by a reflecting plate or the like so that a shadow is rarely generated. The shadowless lamp 7 applies light of a predetermined wavelength band including the visible light to illuminate the operative field 101. The shadowless lamp 7 includes an incandescent lamp, a halogen lamp, a LED illumination, or the like. To the shadowless lamp 7, the cutoff filter f7, which is a film filter, is attached. The cutoff filter f7 composes a short-pass filter which shuts off the light equal to or higher than the peak wavelength band of the fluorescence emission of the affected part 105.

The fluorescent lamp 8 is a fluorescent lamp generating white light and illuminates an entire operating room including the operative field 101. To the fluorescent lamp 8, the fluorescence cutoff filter f8, which is a film filter, is attached. The fluorescence cutoff filter f8 is a short-pass filter which shut off the light equal to or higher than the peak wavelength band of the fluorescence emission of the affected part 105. Instead of the fluorescent lamp 8, an incandescent lamp or a LED illumination may be used.

Fig. 13 is a diagram for explaining filtered wavelength bands of various lights of the medical operation support system according to the fourth embodiment. Fig. 13 shows the wavelength bands of the fluorescent light and the excitation light of indocyanine green that is the photosensitive substance contained in the subject as well as the light from the external light source such as the shadowless lamp 7 and the fluorescent lamp 8. The light from the external light source includes the wavelength band components of the near-infrared region as well as the visible light region because of the characteristics of the light source. Therefore, the external light partially overlaps with the fluorescent light of indocyanine green in a certain wavelength band. For example, a spectrum of a fluorescent lamp generating white light includes a wavelength band component of a near-infrared region.

In this regard, the medical operation support system 200 uses the cutoff filters f7, f8 to shut off the same wavelength band component as the fluorescence emission of the affected part 105 out of the external light. Specifically, the cutoff filters f7, f8 are used for transmitting the wavelength band component less than the wavelength of 800 nm and shutting off the wavelength band component of the wavelength of 800 nm or more out of the lights of the shadowless lamp 7 and the fluorescent lamp 8.

As a result, the imaging unit 12 can be restrained from receiving the component of the external light from the shadowless lamp 7 and the fluorescent lamp 8 in the wavelength band overlapping with the wavelength band of the fluorescence emission so as to reduce the detection noise. Additionally, since the fluorescence cutoff filters f7, f8 transmit the wavelength band component less than the wavelength of 800 nm out of the external light, the component of the external light at the peak wavelength of the excitation light can be transmitted along with the visible light so as to increase the efficiency of causing the affected part 105 to emit fluorescence.

### 4-3. Effect and the like

As described above, in this embodiment, as compared to the medical operation support system 100C, the medical operation support system 200 includes the cutoff filters (third filters) f7, f8 that are attached to the shadowless lamp 7 and the fluorescent lamp 8 applying light of a predetermined wavelength band including the visible light to the affected part 105 and that cut off a predetermined wavelength band component including a peak wavelength of fluorescence.

With the above configuration, the imaging unit 12 can be restrained from receiving the component of the external light in the wavelength band overlapping with the fluorescence emission so as to reduce the detection noise.

### (Other Embodiments)

As described above, the first to third embodiments have been described as exemplifications of the techniques disclosed in the present application. However, the in the present disclosure are not limited thereto and are applicable to embodiments with modification, replacement, addition, omission, or the like made as appropriate. The constituent elements described in the first embodiment can be combined to form a new embodiment.

Therefore, other embodiments will hereinafter exemplarily be described.

Although medical application such as surgery is taken as an example in the description of the first to fourth embodiments, this is not a limitation of the present invention. For example, the present invention is applicable when an operation must be performed to an object having a visually unrecognizable change in state in a construction site, a mining site, a building site, a material processing factor, or the like.

Specifically, instead of the medical device of the first embodiment, a fluorescent material is applied to, kneaded in, or poured into an object having a visually unrecognizable change in state in a construction site, a mining site, a building site, a material processing factor, or the like, to form a capture object that is an object to be captured by the imaging unit 12. Concurrently, by storing shape information on the shape of the object into the memory 4, the present invention can be applied as is the case with the first and third embodiments.

In the first embodiment, the image data 51 to 53 of the surgical instruments stored in the memory 4 are described as examples. However, the memory 4 may store the image data of medical devices including the surgical instruments.

Further, the shape information stored in the memory 4 may be image data preliminarily captured by the imaging unit 12, for example. This enables projection of image data more indicative of an actually used device.

Although the shape information of the first embodiment is the image data indicating the shape of the medical device, the shape information is not limited to the medical device and may be image data of an object having a predetermined shape. The image data of an object having a predetermined shape is not limited to image data of the object itself such as a medical device. The shape information may be image data schematically representing the object and may be, for example, a graphic depicting the object or a mark such as an arrow.

In the first embodiment, the projection image is generated and projected for one medical device. However, the control unit 2 may compare the fluorescent image with a plurality of pieces of the shape information at the same time. Thereby, when a plurality of types of surgical instruments is concurrently inserted in the body of the patient 10, each of the medical devices can be discriminated.

In the first embodiment, the control unit 2 replaces the fluorescent image of the captured image data with the image of the image data determined as having similarity, so as to generate the image data for projection. However, the control unit 2 may refer to the determined image data to correct a shape of an unclear fluorescent image in the captured image data, thereby generating the image data for projection.

In the first to third embodiment, the imaging unit 12 is configured to be able to detect all the type of lights that are the visible light, the fluorescent light, and the excitation light; however, the imaging unit 12 may be configured to be able to detect at least the fluorescent light. For example, the imaging unit 12 may include a combination of cameras capable of detecting only the visible light and the fluorescent light or may include a camera capable of detecting only the fluorescent light.

Although the photosensitive substance is exemplified by indocyanine green in the first to third embodiments, other photosensitive substances may be used. For example, porphyrin, luciferin, Aka Lumine (registered trademark), or the like may be used. In this case, the light source 11 applies the excitation lights of the respective excitation wavelength bands of the photosensitive substances and the imaging unit 12 detects the fluorescent images from the detection lights of the wavelength bands of the fluorescence emission of the respective photosensitive substances.

For example, the light source 11 applies blue light having a wavelength near 400 nm to the subject containing porphyrin and the imaging unit 12 detects the fluorescent image of red light having a wavelength near 600 nm. In this case, for example, the projection image may be projected with green light having a wavelength near 500 nm. This facilitates the discrimination between the fluorescence-emitting region and the projection region. Additionally, in this case, only the red light may be transmitted by the transmission filter f2 of the third embodiment and only the blue light may be transmitted by the fluorescence cutoff filter f1.

Although the dichroic mirror of the dichroic mirror portion 14 is used for matching the imaging optical axis Z2 with the projection optical axis Z3' in the second to fourth embodiments, the dichroic mirror may not be used. For example, a half mirror or a polarizing plate may be used for matching the imaging optical axis Z2 with the projection optical axis Z3'.

Although the transmission filter f2 is a film filter built into the imaging unit 12 in the third and fourth embodiments, the transmission filter f2 may be a film filter attached to a surface of a light-receiving portion of the imaging unit 12.

Although the cutoff filters f1, f7, f8 and the transmission filter f2 are film filters in the third and fourth embodiments, the filters may not be film filters. For example, the filters may be optical filters made of optical glass.

As above, the embodiments have been described as exemplifications of the techniques disclosed in The present disclosure. In this regard, the accompanying drawings and the detailed description are provided.

Therefore, the components described in the accompanying drawings and the detailed description may include not only the components essential for solving the problem but also components not essential for solving the problem so as to exemplarily describing the techniques. Therefore, even though those non-essential components are included in the accompanying drawings and the detailed description, these non-essential components should not immediately be recognized as being essential.

Since the embodiments described above are intended to exemplarily describe the techniques of the present disclosure, various modifications, replacements, additions, and omissions can be made within the claims and the scope equivalent thereto.

### INDUSTRIAL APPLICABILITY

The projection system of the present disclosure is applicable when an operation is performed to an object having a visually unrecognizable change in state for medical application or in a construction site, a mining site, a building site, a material processing factor, or the like.

### EXPLANATIONS OF REFERENCE NUMERALS

- 1: imaging/projecting apparatus
- 11: light source
- 12: imaging unit
- 2: control unit
- 13: projecting unit
- 4: memory
- 10: patient
- 20: medical device
- 100, 100A, 100B, 100C, 200: medical operation support system

### It follows a list of further embodiments of the invention:

Embodiment 1 A projection system comprising:
   an excitation light source for applying to a subject light of a predetermined wavelength band including excitation light;
   an imaging unit for capturing a fluorescent image resulting from fluorescence emission of the subject;
   a control unit for generating image data for projection based on the fluorescent image captured by the imaging unit; and
   a projecting unit for projecting a projection image based on the image data for projection onto the subject with visible light, wherein
   in the projection system, an optical axis of light incident on the imaging unit is adjusted to be matched with an optical axis of light exiting from the projecting unit.
Embodiment 2 The projection system with the features of embodiment 1, further comprising a dichroic mirror for separately guiding light of a wavelength band of fluorescence emitted by the subject and visible light, wherein
   the dichroic mirror is disposed to match the optical axis of the light incident on the imaging unit with the optical axis of the light exiting from the projecting unit.
Embodiment 3 The projection system with the features of embodiment 1 or 2, further comprising
   a first filter for cutting off a predetermined wavelength band component including a peak wavelength of the excitation light out of the light incident on the imaging unit, and
   a second filter for cutting off a predetermined wavelength band component including a peak wavelength of the fluorescence out of the light applied by the excitation light source.
Embodiment 4 The projection system with the features of one of embodiment 1 to 3, comprising a third filter attached to an external light source for emitting to the subject a light of a predetermined wavelength band including visible light, the third filter for cutting off a predetermined wavelength band component including a peak wavelength of the fluorescence.
Embodiment 5 The projection system with the features of one of embodiment 1 to 4, wherein
   the control unit corrects the image data for projection in accordance with a difference between the fluorescent image captured by the imaging unit and the projection image so that an image of a region of the fluorescence emission matches with the projection image.
Embodiment 6 The projection system with the features of one of embodiment 1 to 4, further comprising a storage unit for storing a plurality of pieces of shape information that is image data of an object having a predetermined shape, wherein
   the control unit selects a piece of the shape information based on a degree of similarity to the fluorescent image captured by the imaging unit, from the plurality of pieces of the shape information stored in the storage unit, and wherein
   the control unit generates the image data for projection such that an image having a shape indicated by the selected piece of the shape information is projected onto a region of the fluorescence emission.
Embodiment 7 The projection system with the features of one of embodiment 1 to 6, wherein
   the subject includes an affected part of a patient or a medical device.

## Claims

1. A projection system (100C) comprising:
an excitation light source (11) for applying to a subject (105) light of a predetermined wavelength band including excitation light;
an imaging unit (12) for capturing a fluorescent image resulting from fluorescence emission of the subject (105);
a control unit (2) for generating image data for projection based on the fluorescent image captured by the imaging unit (12); and
a projecting unit (13) for projecting a projection image based on the image data for projection onto the subject (105) with visible light;
a dichroic mirror (14) for transmitting light of a wavelength band of fluorescence emitted by the object (10) and reflecting the visible light from the projecting unit, wherein
the dichroic mirror (14) is disposed to match an optical axis (Z2) of light incident on the imaging unit with an optical axis (Z3') of light exiting from the projecting unit (13),
**characterized in that** the projection system (100C) further comprises a cutoff filter (f1) attached onto the excitation light source (11) for cutting off a predetermined wavelength band component including a peak wavelength of the fluorescence out of the light applied by the excitation light source (11).

2. The projection system (100C) according to claim 1, the excitation light source (11) is disposed to surround both of the matched optical axes (Z2, Z3').

3. The projection system (100C, 200) according to claim 1 or 2, comprising a further filter (f7, f8) attached to an external light source (7, 8) for emitting to the subject (105) a light of a predetermined wavelength band including visible light, the further filter (f7, f8) for cutting off a predetermined wavelength band component including a peak wavelength of the fluorescence.

4. The projection system (100C) according to any one of claims 1 to 3, wherein
the imaging unit (12) is configured to incorporate an imaging optical system therein, and the projecting unit (13) is configured to incorporate a projection optical system therein.

5. The projection system (100C) according to any one of claims 1 to 4, wherein
the control unit (2) corrects the image data for projection in accordance with a difference between the fluorescent image captured by the imaging unit (12) and the projection image (13) so that an image of a region of the fluorescence emission matches with the projection image.

6. The projection system (100C) according to any one of claims 1 to 5, further comprising a storage unit (4) for storing a plurality of pieces of shape information that is image data of an object having a predetermined shape, wherein
the control unit (2) selects a piece of the shape information based on a degree of similarity to the fluorescent image captured by the imaging unit (12), from the plurality of pieces of the shape information stored in the storage unit (4), and wherein
the control unit (2) generates the image data for projection such that an image having a shape indicated by the selected piece of the shape information is projected onto a region of the fluorescence emission.

7. The projection system (100C) according to any one of claims 1 to 6, wherein
the subject (105) includes an affected part (105) of a patient (10) or a medical device (20).
